# NOUVEAU FASCICULE DE BREVET EUROPEEN

(11) **EP 1 280 541 B2**
(45) Date de publication et mention de la décision concernant l'opposition: **17.03.2010**
(45) Mention de la délivrance du brevet: 12.04.2006
(21) Numéro de dépôt: 01934089.2
(22) Date de dépôt: 11.05.2001
(51) Int. Cl.: A61K 35/74, A61P 1/00, C12Q 1/68

(54) **UTILISATION DE SOUCHES ACETOGENES HYDROGENOTROPHES POUR LA PREVENTION OU LE TRAITEMENT DU SYNDROME DE L'INTESTIN IRRITABLE**
VERWENDUNG VON AZETOGENISCHEN HYDROGENOTROPHISCHEN STÄMMEN ZUR VORBEUGUNG UND BEHANDLUNG VON COLON IRRITABLE
USE OF HYDROGENOTROPHIC ACETOGENIC STRAINS FOR PREVENTING OR TREATING IRRITABLE BOWEL SYNDROME

(30) Priorité: 11.05.2000 FR 0006009
(43) Date de publication de la demande: 05.02.2003
(73) Titulaire: INSTITUT NATIONAL DE LA RECHERCHE AGRONOMIQUE, F-75007 Paris (FR)
(72) Inventeur: RENAUD, Michel, F-63670 Le Cendre (FR); BERNALIER Annick, F-63670 Laroche-Blanche (FR)
(74) Mandataire: Warcoin, Jacques
(86) Numéro de dépôt international: PCT/FR2001/001426
(87) Numéro de publication internationale: WO 2001/085187

(56) Documents cités:
- DE-A1- 19 826 928
- JOBLIN K N: "Ruminal acetogens and their potential to lower ruminant methane emissions." AUSTRALIAN JOURNAL OF AGRICULTURAL RESEARCH, vol. 50, no. 8, 1999, pages 1307-1313, XP001010439
- BERNALIER A ET AL: "Ruminococcus hydrogenotrophicus sp. nov., a new H-2/CO-2-utilizing acetogenic bacterium isolated from human feces." ARCHIVES OF MICROBIOLOGY, vol. 166, no. 3, 1996, pages 176-183, XP000979148
- KELLER G H & MANAK M M: "DNA Probes" 1994 , STOCKTON PRESS , NEW YORK XP002158943 208660 page 594 -page 596 * Procedure 15.2 *
- BERNALIER A ET AL: "Acetogenesis from H-2 and CO-2 by methane- and non-methane-producing human colonic bacterial communities." FEMS MICROBIOLOGY ECOLOGY, vol. 19, no. 3, 1996, pages 193-202, XP000979130 cité dans la demande
- DURAND M & BERNALIER A: "Reductive acetogenesis in animal and human gut." PHYSIOLOGICAL AND CLINICAL ASPECTS OF SHORT-CHAIN FATTY ACIDS., 1995, pages 107-117, XP000979817 Cambridge University Press ISBN: 0-521-44048-3
- VAN NEVEL C J & DEMEYER D I: "Control of rumen methanogenesis." ENVIRONMENTAL MONITORING AND ASSESSMENT, vol. 42, 1996, pages 73-97, XP000979267
- L. NOLLET ET AL. APPL MICROBIOL BIOTECHNOL vol. 48, 1997, pages 99 - 104
- A. BERNALIER ET AL. CURRENT MICROBIOLOGY vol. 33, 1996, pages 94 - 99
- RIAL D. ROLFE J. NUTR. vol. 130, 2000, pages 396S - 402S

## Description

L'invention concerne l'utilisation de souches bactériennes acétogènes hydrogénotrophes non pathogènes autologues pour la préparation d'une composition destinée au traitement ou à la prévention chez l'homme du syndrome de l'intestin irritable.

La prévalence des troubles digestifs fonctionnels ou colopathies fonctionnelles dans la population occidentale est très élevée puisque l'on estime qu'ils touchent environ 25% à 30% de la population adulte. De plus, ces troubles digestifs représentent une des causes majeures de consultation en gastro-entérologie (environ 50% des consultations). Les symptômes de ces troubles intestinaux sont divers comme la modification du transit intestinal, le météorisme, les douleurs abdominales, le ballonnement. L'origine de ces troubles fonctionnels reste pour le moment mal définie mais l'on estime que les gaz produits lors de la digestion dans le côlon jouent un rôle important dans la genèse de certains symptômes comme l'excès de flatulences, la distension abdominale (ballonnement) et les douleurs associées. Certains traitements ont été proposés comme le charbon actif, la simethicone, la smectite, des antispasmodiques ainsi que certains compléments alimentaires à base de ferments *(Saccharomyces cerevisiae, Bifidobacterium, Lactobacillus),* de plantes ou de fibres (oligofructose,, fenouil, algues, avoine, agrumes...) ou de structure minérale (Octalite...). Ces traitements présentent toutefois une faible efficacité sur les symptômes liés à la formation des gaz au niveau du côlon, et n'agissent pas de manière sélective. La présente invention se propose de remédier aux inconvénients de l'art antérieur, tant sur le plan du traitement que sur le plan de la prévention de l'inconfort digestif associé aux productions de gaz colique.

Pour ce faire, l'invention s'appuie à la fois sur les caractéristiques physiologiques des bactéries acétogènes hydrogénotrophes, à savoir leur capacité à réduire le volume total des gaz fermentaires digestifs (H₂ et CO₂) et sur leur diversité nutritionnelle qui leur confère un avantage écologique important dans l'écosystème digestif par rapport aux autres microorganismes hydrogénotrophes.

Chez l'homme, les glucides alimentaires qui échappent à la digestion et l'absorption dans l'intestin grêle arrivent dans le côlon où ils sont fermentés par une microflore complexe. Cette dégradation anaérobie de la matière organique produit des métabolites terminaux sous forme d'acides gras volatiles ayant des propriétés métaboliques (acétate, propionate) ou trophiques (butyrate) ainsi que des gaz (H₂, CO₂ et, chez certains individus, CH₄).

Parmi ces gaz fermentaires, H₂ joue un rôle important dans le maintien et l'efficacité de la dégradation de la matière organique dans le côlon humain. Une partie de H₂ est éliminée par les voies respiratoires et rectales, mais la plus grande fraction de ce gaz est réutilisée *in situ* par la flore intestinale. Cette dernière, appelée flore hydrogénotrophe, est composée de bactéries acétogènes, de bactéries sulfato-réductrices et d'archæa méthanogènes.

On trouve les bactéries sulfato-réductrices dans la microflore digestive de tous les individus (Pochart et al. (1992) FEMS Microbiol. Lett. 98 p225). Elles synthétisent H₂S qui est un produit potentiellement toxique pour les cellules eucaryotes, et qui serait impliqué dans certaines maladies du système digestif, en particulier les colites ulcéreuses (Roediger et al. (1993), Gastroenterology, 104, p802).

Les archæa méthanogènes produisent du CH₄ qui est un gaz non toxique. Cette production de méthane n'est observée que chez une fraction de la population humaine (environ 21% des indiens adultes, 95% de la population rurale d'adolescents d'Afrique noire et 40% de la population occidentale) et son élimination se fait par voie respiratoire et dans les flatulences (Segal et al. (1988) Gut 29 p608; Pochart et al. (1992) FEMS Microbiol. Lett. 98 p225). Ces individus, appelés méthano-excréteurs, hébergent une population d'archæa méthanogènes très importante (>10⁸/g d'extrait fécal sec) (Durand et al. (1996) in: Mälkki Y and Cummings JH (edsOfficial Publications of the European Communities, p58). Chez ces sujets, la méthanogénèse est la principale voie d'élimination de H₂.

Les individus non méthano-excréteurs réutilisent H₂ par des mécanismes alternatifs, parmi lesquels l'acétogénèse réductrice. Cette voie constitue un processus métabolique majeur d'utilisation de H₂ chez les non méthano-excréteurs. Des études ont en effet montré que la microflore fécale des sujets non méthano-excréteurs métabolise principalement H₂ et CO₂ en acétate, alors que celle des sujets méthano-excréteurs utilisent H₂ et CO₂ pour former du méthane (Lajoie et al. (1988) Appl. Environ. Microbiol. 54 p2733 ; Bemalier et al. (1996) FEMS Microbiol. Ecol. 19 p193). En parallèle, Doré et al. (1995 FEMS Microbiol. Ecol. 17 p279) ont montré l'existence d'une corrélation négative entre le nombre d'archæa méthanogènes et celui des bactéries acétogènes dans le côlon humain. Les individus non méthano-excréteurs hébergent donc peu ou pas d'archæa méthanogènes dans le côlon, ce qui permettrait une expression maximale de leur activité acétogène (Lajoie et al. (1988) Appl. Environ. Microbiol. 54 p2733 ; Bernalier et al. (1996) FEMS Microbiol. Ecol. 19 p193).

La flore acétogène hydrogénotrophe se caractérise par une grande diversité taxonomique. Elle est composée d'espèces bactériennes appartenant en particulier aux genres *Clostridium, Ruminococcus* et *Streptococcus* (Bernalier et al. (1996) Curr. Microbiol. 33 p94) ainsi que de certaines espèces du genre *Eubacterium* (Schink (1994) in: Drake HL (ed) Acetogenesis. New-york: Chapman and Hall p197).

Par « bactéries acétogènes hydrogénotrophes » , on entend les espèces bactériennes utilisant la voie réductrice de synthèse de l'acétate (ou voie de Wood-Ljungdahl) pour produire ce métabolite lors de leur croissance autotrophe à partir de H₂/CO₂ ainsi que lors de leur croissance hétérotrophe, à partir d'un substrat organique. Ces bactéries acétogènes hydrogénotrophes présentent en effet une grande capacité nutritionnelle et sont capables, outre d'utiliser H₂/CO₂, de fermenter un nombre important d'oses et de composés organiques (Bernalier et al (1996), Curr. Microbiol., 33 p94).

Les souches bactériennes acétogènes hydrogénotrophes selon l'invention produisent des acides gras à chaîne courte (AGCC), en particulier de l'acétate, à partir des gaz H₂ et CO₂. Cette production d'AGCC présente un avantage physiologique pour l'hôte, comme la prévention (protection) ou le traitement de pathologies diverses (voir ci-dessous).

La présence simultanée de composés organiques et de H₂/CO₂ dans le milieu de culture (conditions équivalentes à celles rencontrées dans le côlon humain ) peut se traduire par une utilisation simultanée des deux substrats par la souche acétogène hydrogénotrophe (Breznak and Blum (1991), Arch. Microbiol., 156 p105). Ce phénomène appelé mixotrophie, permet à la bactérie d'avoir un rendement énergétique plus élevé et donc de croître plus rapidement.

Cette aptitude à consommer H₂/CO₂ associée à la capacité d'utilisation d'un grand nombre de substrats organiques ainsi que la capacité à croître par mixotrophie, confère donc un avantage écologique important aux bactéries acétogènes par rapport aux populations de méthanogènes qui n'utilisent qu'un nombre restreint de substrats (H₂, formate), et sulfatoréductrices dépendantes de la présence de sulfate pour leur métabolisme de H₂.

Il a été montré que l'utilisation de certaines préparations probiotiques, contenant des bactéries telles que des bactéries propioniques, lactobacilles, et/ou bifidobactéries, permet de modifier la flore colique de certains patients (Bougle et al. (1999) Scand. J. Gastroenterol. 34 p144 ; Venturi et al. (1999) Aliment. Pharmacol. Ther. 13 p1103)

L'utilisation des bactéries acétogènes en tant que probiotiques comme définis par Fuller (1989, J. Appl. Bact., 66 p365), dans des préparations qui peuvent être utilisées en tant qu'alicaments ou en tant que suppléments alimentaires, s'avère donc être une voie d'intérêt particulièrement novatrice, car leur capacité à métaboliser H₂/CO₂ permettrait d'optimiser les fermentations coliques en diminuant le volume total des gaz fermentaires et en produisant de l'acétate, source d'énergie métabolisable par l'hôte. La réduction des gaz digestifs serait ainsi un moyen efficace de prévention et/ou de traitement des troubles digestifs liés à l'accumulation de ces gaz.

Le domaine de la présente invention est l'utilisation de souches acétogènes hydrogénotrophes non pathogènes pour réguler les susdits désordres digestifs et/ou moduler l'équilibre de la flore microbienne chez un mammifère.

Les mammifères peuvent être les mammifères monogastriques, par opposition aux mammifères polygastriques comme les ruminants. On entend en particulier les félins et canins, en particulier les mammifères domestiques (chats et chiens), ainsi que l'homme.

Par « non pathogène », on entend une espèce microbienne pour laquelle aucune pathologie de l'hôte associée à sa présence n'a pu être mise en évidence (souche GRAS = Generally Recognized As Safe).

Une telle utilisation peut être envisagée de différentes façons. La présente invention concerne une utilisation prophylactique ou thérapeutique, afin de prévenir et/ou traiter certains troubles de l'appareil digestif. Cette prévention et/ou traitement peut s'effectuer par l'intermédiaire d'une régulation des gaz produits dans le côlon, grâce à la modulation de la flore microbienne. Une utilisation de ce type peut être envisagée sous la direction d'un médecin ou d'un professionnel de santé. Dans ce cas, le dosage, la durée du traitement, ainsi qu'une association éventuelle de la souche acétogène hydrogénotrophe non pathogène avec d'autres principes actifs efficaces pour la prévention et/ou le traitement des troubles digestifs visés, sont décidés par le professionnel de santé. Une telle utilisation peut également nécessiter un suivi de la souche acétogène hydrogénotrophe non pathogène par un procédé d'analyse, tel que défini plus loin.

En effet, les troubles digestifs visés par la présente invention affectent la qualité de vie des patients qui en sont atteints. Le degré d'inconfort digestif engendré et/ou la capacité de chacun à supporter ces troubles expliquent que les personnes atteintes consultent ou non un médecin.

En particulier, l'invention concerne l'utilisation de souches acétogènes hydrogénotrophes pour la préparation d'une composition pour les applications suivantes :
(1) la prévention et/ou le traitement des troubles fonctionnels digestifs,
(2) la modulation de l'équilibre de la flore microbienne colique en favorisant avantageusement l'activité de la flore bactérienne acétogène, en particulier au détriment des flores bactériennes méthanogènes et sulfato-réductrices.

Ce dernier point a pour intérët :
(1) de diminuer la formation du gaz CH₄,
(2) de diminuer la production de H₂S, produit toxique, impliqué dans l'initiation et/ou le développement de pathologies digestives,
(3) de favoriser la production de métabolites sains pour l'hôte.

En particulier, on utilise une souche du genre *Ruminococcus, Clostridium* ou *Streptococcus,* de façon préférée *Ruminococcus hydrogenotrophicus.*

Ainsi, la présente invention concerne l'utilisation d'au moins une souche bactérienne acétogène hydrogénotrophe non pathogène autologue pour la préparation d'une composition destinée à la prévention et/ou au traitement du syndrome de l'intestin irritable en réduisant la formation de gaz potentiellement toxiques chez l'homme.

Ladite réduction s'effectue par la diminution du taux d'hydrogène gazeux (H₂) et/ou de dioxyde de carbone gazeux (CO₂) produit lors des fermentations digestives.

Ladite réduction peut également s'effectuer par l'augmentation de l'activité de la flore colique acétogène au détriment de la flore méthanogène et/ou sulfato-réductrice.

Les flatulences excessives, le météorisme, les ballonnements, les douleurs abdominales sont des critères majeurs caractérisant le syndrome de l'intestin irritable.

Il est également décrit une composition alimentaire susceptible d'être utilisée dans l'élaboration de nouveaux aliments ou ingrédients alimentaires tels que définis dans le règlement CE n°258/97, et notamment dans la fabrication d'aliments fonctionnels. Un aliment peut être considéré comme fonctionnel si l'on démontre de façon satisfaisante qu'il exerce un effet bénéfique sur une ou plusieurs fonctions cibls dans l'organisme, au delà des effets nutritionnels habituels, en améliorant l'état de santé et de bien être et/ou en réduisant le risque d'une maladie (Diplock et al. Scientific concepts of fonctional foods in Europe : consensus document, British Journal of Nutrition, 1999, 81, S1-S27).

La composition ainsi décrite peut en particulier constituer un probiotique conditionné par exemple sous forme de capsule ou de gélule.

On peut ainsi utiliser une telle composition alimentaire qui contient une souche acétogène hydrogénotrope non pathogène, et qui apporte un bien-être à l'utilisateur par la réduction de l'inconfort digestif

On décrit également une composition pharmaceutique, associée en outre à un support pharmaceutiquement acceptable, qui peut comprendre des excipients. Son mode d'administration s'effectue de préférence par voie orale ou directement *in situ,* en particulier par coloscopie, ou par voie rectale par l'intermédiaire de suppositoires.

Une telle composition pharmaceutique comprend en outre au moins un autre agent actif contre au moins une des pathologies visées.

La susdite composition pharmaceutique ou alimentaire peut être administrée par voie orale, sous forme de gélules, de capsules, de comprimés, de poudres, de granules ou de solutions ou suspensions orales. On peut mélanger la au moins une souche bactérienne avec des excipients classiques tels que la gélatine, l'amidon, le lactose, le stéarate de magnésium, le talc, la gomme arabique et analogues. Il peut également être avantageux d'utiliser des excipients moins classiques, qui permettent d'augmenter la capacité de la au moins une souche bactérienne utilisée à être active dans le côlon. Par exemple, on peut ajouter du cellobiose, du maltose, du mannose, de la salicine, du tréhalose, de l'amygdaline, de l'arabinose, du mélobiose, du rhamnose et/ou du xylose. Cette liste n'est pas exhaustive et les substrats sont choisis et adaptés en fonction de la souche considérée. Ces substrats peuvent favoriser la croissance hétérotrophe et/ou mixotrophe de la au moins une souche acétogène présente dans la composition.

Ainsi, de façon préférée, la composition, préparée dans le cadre de l'invention comprend au moins un additif qui favorise l'activité de la au moins une souche dans l'environnement digestif

Dans un mode de réalisation particulier de l'invention, la au moins une souche bactérienne acétogène hydrogénotrophe non pathogène présente dans la composition est administrée sous une forme lui permettant d'être active dans le côlon. En particulier, il est nécessaire que la au moins une souche bactérienne acétogène hydrogénotrophe non pathogène soit vivante, ou viable dans le tractus digestif, et en particulier le côlon. On peut également, après production de la au moins une souche bactérienne acétogène hydrogénotrophe non pathogène et en fonction des procédés de production, maintenir celle-ci dans des conditions de conditionnement anaérobie, afin de lui permettre de rester viable.

Dans un mode de réalisation préféré, la au moins une souche bactérienne acétogène hydrogénotrophe non pathogène est conditionnée dans un environnement anaérobie, c'est à dire que celle-ci est conditionnée dans une atmosphère exempt d'oxygène.

La au moins une souche bactérienne acétogène hydrogénotrophe non pathogène présente dans la composition est une souche autologue dudit mammifère, c'est à dire qu'elle peut étre isolée dans l'appareil digestif, en particulier dans les fèces d'autres mammifères appartenant au même genre.

D'une manière préférée, la au moins une souche bactérienne acétogène hydrogénotrophe non pathogène appartient au genre *Ruminococcus,* de manière encore plus préférée à l'espèce *Ruminococcus hydrogenotrophicus.* On peut également utiliser d'autres bactéries acétogènes hydrogénotrophes, en particulier des bactéries du genre *Streptococcus* ou *Clostridium,* en particulier *Clostridium coccoides.*

On décrit également un procédé de suivi spécifique de la souche bactérienne acétogène hydrogénotrophe non pathogène dans le tractus digestif d'un mammifère, après son utilisation telle que définie ci-dessus, comprenant les étapes suivantes :
a. on définit une séquence nucléotidique spécifique (sonde) de la souche acétogène hydrogénotrophe non pathogène que l'on désire détecter ;
b. on effectue la détection et/ou quantification de ladite souche par hybridation de la sonde avec de l'acide nucléique total extrait de la flore fécale ou avec les bactéries fécales fixées sur une lame.

Afin de mettre en oeuvre un tel procédé de suivi, on peut développer des trousses de diagnostic. De telles trousses contiennent en particulier un « étalon » afin de pouvoir évaluer la quantité de bactéries dans les fèces.

L'homme du métier sait définir une séquence spécifique, qui ne s'hybride pas avec l'ADN d'autres bactéries. De même, l'homme du métier choisira l'hybridation sur membrane ou l'hybridation *in situ* en fonction des moyens dont il dispose, et de la précision recherchée.

De préférence, l'acide nucléique détecté est l'ADN bactérien total, mais peut également être un mélange d'ADN ou d'ARN, ou l'ARN bactérien seul.

On peut également étudier la présence de la au moins une souche bactérienne acétogène hydrogénotrophe non pathogène, par d'autres méthodes. Une détection des acides nucléiques (ADN et/ou ARN) de la au moins une souche bactérienne acétogène hydrogénotrophe non pathogène dans les fèces du mammifère, en particulier par PCR, RT-PCR ou par hybridation avec des sondes spécifiques (Southern ou Northern) permettra de détecter la présence de ladite souche, et éventuellement l'expression de certains gènes. Une séquence spécifique avantageuse peut être choisie dans la séquence du gène codant pour l'ARNr 16S, en particulier une zone qui n'est pas présente sur les autres espèces de la flore colique.

On décrit également un procédé de production d'une souche bactérienne acétogène hydrogénotrophe non pathogène pour son utilisation telle que définie ci-dessus, caractérisé en ce qu'il comprend les étapes suivantes :
a. on effectue la croissance de la souche sur un milieu adapté, en condition d'anaérobiose stricte, en présence d'un substrat carboné et/ou de H₂/CO₂ comme source d'énergie ;
b. on récupère les cellules bactériennes ;
c. on conditionne les cellules bactériennes suivant la forme galénique choisie.

La croissance de la souche aura lieu de préférence dans un milieu AC21 modifié (décrit dans l'exemple 1), à 37 °C, dans un fermenteur. Le substrat carboné peut être le glucose.

Une méthode préférée de récupération des cellules bactériennes est la centrifugation, par exemple entre 10000g et 15000g, avantageusement 12000g, pendant 15 à 20 minutes. L'homme du métier sait optimiser ces paramètres.

On peut avantageusement laver les bactéries entre les étapes b et c, en particulier dans un tampon phosphate anaérobie, en resuspendant les cellules, agitant et une nouvelle étape de centrifugation.

Le culot bactérien lavé ou non est conditionné en fonction de la forme galénique choisie. Une méthode avantageuse est la lyophilisation.

Les exemples qui suivent permettent d'illustrer l'invention, mais ne doivent pas pour autant être considérés comme limitatifs.

### DESCRIPTION DES FIGURES

Figure 1 : Influence d'un traitement de 14 jours avec *Ruminococcus hydrogenotrophicus* sur les quantités d'hydrogène excrété par des rats à flore humaine en situation nutritionnelle normale.
Figure 2 : Influence d'un traitement de 14 jours avec *Ruminococcus hydrogenotrophicus* sur les quantités d'hydrogène excrété par des rats à flore humaine après administration de lactulose

### EXEMPLES

### Exemple 1 : Isolement des micro-organismes

On utilise des échantillons fécaux humains provenant de volontaires sains, non méthano-excréteurs. Les individus sont considérés comme non méthano-excréteurs lorsque leur taux de méthane expiré n'excède pas plus de 1 ppm celui de l'air ambiant qui est de 1,8 ppm (Bond et al. (1970) Gastroenterology 58 p 1035), et que le nombre de méthanogènes contenu dans leurs extraits fécaux est inférieur à 10⁷/g d'extrait fécal (Bernalier et al. (1996) Arch Microbiol. 166 p176). Le taux de méthane est déterminé à l'aide d'un chromatographe équipé d'un détecteur à ionisation de flamme. Les échantillons fécaux fraîchement prélevés sont conservés à 4°C sous anaérobiose stricte pendant 10 heures au maximum.

L'enrichissement, l'isolement et la culture des micro-organismes sont effectués sur un milieu semi-synthétique, le milieu AC-21 modifié (Breznak et al. (1988) Arch. Microbiol. 150 p282) sous anaérobiose stricte (Hungate (1969) in: Norris JR and Gibbons DW (eds) Methods in microbiology Vol. 3B, New-york: Academic Press p117). La composition par litre du milieu semi-synthétique est la suivante:

| | |
|---|---|
| KH₂PO₄ | 0,2 g |
| NH₄Cl | 0,25 g |
| KCl | 0,5 g |
| CaCl₂.2H₂O | 0,15 g |
| MgCl₂.6H₂O | 0,6 g |
| Na₂SO₄ | 0,1 g |
| Extrait de levure | 0,5 g |
| Tryptone | 2 g |
| Solution d'oligo-éléments | 1 ml |
| Solution Tungstate-Sélénium | 0,1 ml |
| Solution de vitamines | 5 ml |
| Solution de Resazurine (1%, w/v) | 1 ml |
| NaHCO₃ (1 M) | 30 ml |
| Solution réductrice Cystéine/Sulfure (1,25%/1,25%, w/v) | 20 ml |

La solution d'oligo-éléments est préparée d'après Widdel et al. (1983, Arch. Microbiol., 134, p286), et la solution de vitamines est préparée d'après Greening et Leedle (1989, Arch. Microbiol., 151, p399). La solution de Tungstate-Sélénium possède la composition suivante: 0,1 mM Na₂WO₄ et 0,1 mM Na₂SeO₃, dans NaOH 20 mM.

Le milieu semi-synthétique est solidifié par l'ajout d'un agent gélifiant (Agar à 2%). Après inoculation, le gaz du milieu de culture est remplacé par H₂/CO₂ ou N₂/CO₂ en fonction du test envisagé.

Le milieu de dilution est un milieu anaérobie purement minéral (Doré et al. (1995) FEMS Microbiol. Lett. 130 p7). A partir d'un échantillon fécal, une suspension mère est réalisée (diluée au dixième, w/v). Une série de dilutions décimales est effectuée à partir de la suspension mère. Les dilutions ainsi réalisées sont inoculées dans le milieu semi-synthétique liquide contenant H₂/CO₂ (60: 40, v/v, 202kPa) comme seule source d'énergie (Doré et al. (1995) FEMS Microbiol. Lett. 130 p7 ; Bemalier et al. (1996) FEMS Micxobiol. Ecol. 19 p193 ; Bemalier et al. (1996) Curr. Microbiol. 33 p94). Après incubation à 37°C pendant 20 jours, les enrichissements sont obtenus à partir des tubes de dilution les plus élevées et présentant une croissance bactérienne, une consommation de gaz et une production stoechiométrique d'acétate les plus élevées (Bernalier et al. (1996) Curr. Microbiol. 33 p94). La diminution de la pression de gaz dans les cultures est déterminée par mesure directe de la pression partielle avec un manomètre de type Capshuelic (Dwyer, Instruments, Michigan City, Mich., USA). Après trois transferts des cultures enrichies, des colonies bactériennes sont isolées par la méthode des Roll-tubes (Hungate (1969) in: Norris JR and Gibbons DW (eds) Methods in microbiology Vol. 3B, New-york: Academic Press p117) contenant un milieu homologue gelosé et H₂/CO₂ comme source d'énergie. Après 20 jours d'incubation à 39°C, les colonies sont transférées dans des milieux liquides. La purification des cultures est obtenue après 3 à 5 transferts successifs en Roll-tubes, au terme desquels la pureté des cultures est déterminée par microscopie à contraste de phase, après coloration de Gram.

L-ADN total des souches acétogènes hydrogénotrophes isolées est extrait par la méthode de Lawson et al ( 1989, FEMS Microbiol. Lett. 65 p41). Le gène codant pour l'ARN ribosomal 16S est ensuite amplifié par PCR à l'aide des primers universels ARI et pH. Les produits de PRC sont purifiés puis séquencés à l'aide d'un kit de séquence « Dye-Dideoxy Terminator cycle Séquence » et d'un séquenceur automatique Applied Biosystem modèle 373A. La recherche d'homologie de séquence ARNr 16S entre les souches acétogénes isolées et les autres espèces est effectuée grâce au programme FASTA, les bases de données de séquences étant celles de l'EMBL et du RDP, et en utilisant les paramètres de base suggérés. Les alignements de séquence sont vérifiés manuellement.

Par cette méthode, une bactérie du genre *Ruminococcus* a pu être isolée et identifiée à l'espèce *Ruminococcus hydrogenotrophicus.* Cette bactérie a été déposée à la Deutsche Sammlung von Mikroorganismen (Mascheroder Weg 1b, 38124 Braunscheig, Allemagne) sous le numéro DSM 10507, et également sous le numéro DSM 14294 le 10 mai 2001 (Traité de Budapest).

### Exemple 2 : Etude des caractères généraux

1- Le type membranaire des bactéries est déterminé par coloration Gram (méthode conventionnelle) et par le test KOH d'après Buck (1982 App. Environ. Microbiol. 44 p992).
2- L' activité catalase est mesurée en mélangeant 1 ml de suspension bactérienne avec quelques gouttes de H₂O₂ (30%). La production de bulles de gaz se dégageant de façon plus ou moins intense indique la présence d'une catalase.
3- L'activité cytochrome oxydase est étudiée en plaçant une colonie bactérienne sur un disque de papier filtre saturé avec du diméthyl-p-phénylène diamine. Une coloration rouge pourpre survenant immédiatement sur le disque indique que le test est positif.
4- Les caractéristiques morphologiques des cultures sont étudiées par microscopie à contraste de phase et par microscopie électronique après coloration négative à l'uranyl-acétate 2%. Les cellules sont pré-fixées dans du glutaraldéhyde 2% (15 h à 4°C) puis fixées avec le OsO₄ 2% (4°C, pendant 15 h au maximum). Les cellules sont ensuite incluses dans de l'EPPON-812 et les blocs sont très finement coupés. Ces coupes sont contrastées avec l'uranyl-acétate, trempées dans le sel d'acétate et observées à l'aide d'un microscope électronique à transmission (Philips 400).
5- Le type respiratoire des bactéries est étudié par détermination de la croissance en présence ou en absence de O₂.
6- L'effet des variations de pH sur la croissance bactérienne est étudié en modifiant le ratio CO₂/NaHCO₃ du milieu semi-synthétique (Costilow (1981) American Society for Microbiology, Washington DC p66). Les mesures de la croissance bactérienne (DO600) sont réalisées après 24 ou 48 h d'incubation à 37°C. La recherche de la température optimale de croissance est effectuée sur milieu semi-synthétique contenant du glucose et la croissance est observée à des températures variant de 20 à 45°C. Chaque expérience est réalisée en triple. La croissance bactérienne est suivie à l'aide d'un spectrophotomètre, Spectronic 20D (Bioblock Scientific, Illkirch, France).

On a trouvé que *Ruminococcus hydrogenotrophicus* DSM 10507 est un coccobacille à Gram positif, non sporulé et anaérobie stricte. La coloration négative révèle l'absence de flagelles. Les cellules bactériennes sont isolées ou en paire. La souche ne possède pas de catalase ni de cytochrome oxydase. La température et le pH de croissance optimale sont respectivement de 35-37°C et 6,6. Les colonies sont translucides, de couleur blanche à légèrement brune avec des bords réguliers, circulaires et de diamètre variant entre 1 et 2 mm.

### Exemple 3 : Test de croissance, détermination des activités acétogènes

L'aptitude de différentes souches bactériennes à métaboliser H₂/CO₂ et à former de l'acétate est étudiée. Les souches de *Ruminococcus hydrogenotrophicus* DSM 10507, ainsi que celles taxonomiquement proches *Ruminococcus productus* DSM 3507, *Ruminococcus productus* DSM 2950, *Ruminococcus hansenii* DSM 20583, et *Clostridium coccoides* DSM 935 (numéros DSM correspondant aux numéros des organismes déposés à la Deutsche Sammlung von Mikroorganismen) sont incubées sur le milieu AC-21 modifié en présence de H₂/CO₂ (60: 40, v/v, 202 kPa) comme source d'énergie. Des cultures témoin sont incubées sous N₂/CO₂ (60: 40, v/v, 150 kPa). Trois cultures (1 témoin et 2 essais) sont effectuées pour chaque souche bactérienne. La croissance autotrophe est déterminée par incubation pendant 96 h en présence de H₂/CO₂ (60: 40, v/v, 202 kPa). La production d'acétate est mesurée par un test enzymatique (Boehringer Mannheim, Meylan, France), après 6 jours d'incubation à 37°C.

La consommation de H₂/CO₂ est mesurée
1) par détermination du volume gazeux consommé
2) par analyse chromatographique (CPG) de la composition de la phase gazeuse.

La croissance hétérotrophe des souches acétogènes (DO₆₀₀) est étudiée par incubation des bactéries pendant 20 h avec du glucose (2 g/l) ou du fructose (2 g/l) comme seule source d'énergie.

La fermentation du glucose est étudiée par l'incubation des cellules à 37°C pendant 20 h sur le milieu semi-synthétique contenant 2 g/l de glucose et une atmosphère composée à 100% de CO₂. A la fin de l'incubation, les acides gras volatiles du surnageant sont dosés par chromatographie, après conversion en dérivés tertiaires du butyldiméthyl (Richardson et al. (1989) Lett. Appl. Microbiol. 9 p5).

On observe que le temps de doublement de *R. hydrogenotrophicus à* 37°C sur milieu AC-21 modifié en présence de H₂/CO₂ (60: 40, v/v, 202 kPa) comme substrat est de 26,4 h. Les temps de doublement de *R. hydrogenotrophicus* à 37°C avec le glucose ou le fructose comme source d'énergie sont d'environ 2 ou 3 h.

On observe que la souche bactérienne étudiée est acétogène : elle présente une croissance autotrophe en présence de H₂/CO₂ et produit de l'acétate comme métabolite majeur (Tableau I). Parmi les espèces taxonomiquement proches, l'activité acétogène (i.e. consommation de H₂/CO₂ et production d'acétate) est retrouvée chez *C*. *coccoides* et beaucoup plus faiblement chez *R. hansenii* et *R. productus.*

*R. hydrogenotrophicus* DM 10507 consomme environ 120mM de H₂ après 96 heures de culture à 37°C (1,25mM de H₂ consommé par heure). La production totale d'acétate est alors égale à 30mM (stoechiométrie : 4 H₂ consommés pour 1 acétate formé).

**Tableau I : Caractéristiques fermentaires des souches cultivées en présence de H2/CO2 ou de glucose comme seule source d'énergie**

| Propriétés | *R.* | *C.* | *R.* | *R.* | *R.* |
|---|---|---|---|---|---|
| | *hydrogenotrophicus* DSM 10507 | *coccoides* DSM 935 | *productus* DSM3507 | *productus* DSM 2950 | *hansenii* DSM 20853 |
| **Utilisation** | ++ | + | - | + | +/- |
| **de H₂/CO₂** | | | | | |
| **PF de** | | | | | |
| **H₂/CO₂** | | | | | |
| Acétate | ++ | + | - | + | +/- |
| Propionate | - | - | - | - | - |
| Butyrate | - | - | - | - | - |
| Lactate | - | - | - | - | - |
| Succinate | - | - | - | - | - |
| Ethanol | - | - | - | - | - |
| **PF du** | | | | | |
| **Glucose** | | | | | |
| Acétate | ++ | + | ++ | ++ | ++ |
| Propionate | - | - | - | - | - |
| Butyrate | - | - | - | - | - |
| Lactate | + | - | - | - | + |
| Succinate | - | ++ | - | - | + |
| Ethanol | + | - | + | + | - |

Symboles : PF, produits de fermentation ; ++, métabolite majoritaire ; +, métabolite non majoritaire ; +/-, métabolite produit en faible quantité ; -, métabolite non produit.

### Exemple 4 : Estimation de la capacité acétogène hydrogénotrophe de R. hydrogenotrophicus par mesure de l'incorporation de ¹³CO₂ dans l'acétate (méthode RMN)

L'incorporation de ¹³CO₂ dans l'acétate est mesurée par RMN, à partir de suspensions cellulaires de *R. hydrogenotrophicus* incubées en présence de H₂. Les bactéries sont cultivées en fiole de 1 1 contenant 250 ml de milieu AC21 tel que décrit dans l'exemple 1 et H₂/CO₂ comme seule source d'énergie. Après croissance à 37°C, les cellules bactériennes sont récupérées par centrifugation (12 000g pendant 20 minutes) et resuspendues dans un tampon phosphate contenant 20 mM de NaH¹³CO₃ (Leclerc et al (1997), Anaerobe, 3, p307). Les suspensions sont incubées pendant 20 heures à 37°C, sous une atmosphère composée de 100% de N₂ (témoin) ou de H₂/N₂ (80/20, V/V), à 101 atm. En fin d'incubation, les suspensions sont à nouveau centrifugées à 12 000g pendant 20 minutes et les surnageants sont récupérés. La production totale d'acétate est mesurée par méthode enzymatique (kit Boehringer-Mannheim). Les métabolites marqués au ¹³C sont analysés par RMN (Bernalier et al, (1996), FEMS Microbiol. Ecol., 19, p193).

Lorsque la bactérie est incubée en présence de H₂, le seul métabolite détecté par RMN du ¹³C est l'acétate. Le ¹³C-acétate est marqué de façon équivalente sur ses groupements méthyle et carboxyle. L'acétate doublement marqué représente 72% de l'acétate marqué total. Ceci confirme que la synthèse d'acétate à partir de H₂ et CO₂ par R. *hydrogenotrophicus* s'effectue selon la voie réductrice de l'acétogénèse.

### Exemple 5 : Détermination des capacités nutritionnelles des bactéries acétogènes

Le métabolisme d'autres substrats organiques par les bactéries acétogènes est évalué en utilisant un milieu AC-21 modifié contenant 5 ou 10 mM de substrat, dans une atmosphère composée de CO₂ à 100%. Le test est considéré comme positif quand la bactérie maintient sa croissance après trois transferts successifs sur un milieu contenant le même substrat, et si la DO₆₀₀ de la culture est au moins égale au double de celle observée avec un milieu semi-synthétique basal (exempt de substrat organique), après 24 h d'incubation à 37°C.

On observe que de nombreux substrats organiques permettent le croissance hétérotrophes des bactéries considérées (Tableau II).

**Tableau II: Croissance de R. hydrogenotrophicus et des espèces taxonomiquement proches en présence de divers substrats organiques**

| Substrat | *R.* | *C.* | *R.* | *R.* | *R* |
|---|---|---|---|---|---|
| | *hydrogenotrophicus* DSM 10507 | *coccoides* DSM 935 | *productus* DSM3507 | *productus* DSM 2950 | *hansenii* DSM 20853 |
| Amidon | - | - | + | + | - |
| Amygdaline | - | + | + | + | +/- |
| Arabinose | - | + | + | + | - |
| Cellobiose | + | + | + | + | - |
| Fructose | + | + | + | + | - |
| Galactose | + | + | + | + | + |
| Inuline | - | - | - | - | + |
| Lactose | + | + | + | + | + |
| Maltose | +/- | + | + | + | + |
| Mannitol | - | + | + | + | - |
| Mannose | + | + | + | + | - |
| Melibiose | +/- | + | + | + | + |
| Raffinose | + | + | + | + | + |
| Rhamnose | - | + | - | - | - |
| Salicine | + | + | + | + | - |
| Sorbitol | - | + | + | + | - |
| Saccharose | - | + | + | + | - |
| Trehalose | + | + | + | + | + |
| Xylose | - | + | + | + | - |

| | | | | | |
|---|---|---|---|---|---|
| Symboles : -, pas de croissance visible ; +/-, faible croissance ; +, bonne croissance. | | | | | |

Par ailleurs, la croissance de différentes souches acétogènes hydrogénotrophes isolées de selles humaines, en présence de divers composés aromatiques comme le vanillate, le cafféate ou le syringeate, est estimée par mesure de la densité optique à 600 nm à l'aide d'un spectrophotomètre Spectronic 20D (l'effet de l'addition de H₂ dans la phase gazeuse de ces cultures est également étudié). La quantité de substrat dégradé est déterminée par HPLC ainsi que la nature des métabolites formés.

La capacité à métaboliser les différents substrats aromatiques testés dépend de la souche acétogène hydrogénotrophe considérée. Les espèces de *Clostridium* sont capables de croître et de dégrader 20% à 30% du cafféate et du syringeate, cette dégradation atteignant 100% lorsque H₂ est additionné à la culture. Une des souches de *Clostridium* ne dégrade le vanillate qu'en présence de H₂ dans la phase gazeuse. Cette souche déméthyle le vanillate en protocatéchuate lors d'une première étape, puis, grâce à H₂, décarboxyle ce composé en catéchol. La souche de *R. hydrogenotrophicus* ne présente qu'une faible activité à l'égard du vanillate, ce métabolisme n'apparaissant pas influencé par la présence de H₂.

La capacité de *R. hydrogenotrophicus* à utiliser 2 substrats organiques non digestibles par les enzymes de l'hôte, les fructo-oligosaccharides (FOS) et le lactulose est également déterminée selon le protocole décrit dans cet exemple [mesure la croissance bactérienne (densité optique à 600 nm) observée en présence de 2g/l de chacun des substrats indigestibles, et maintien de celle-ci après 3 transferts successifs sur le même substrat]. *R. hydrogenotrophicus* s'avère incapable de métaboliser ces 2 substrats.

### Exemple 6 : Caractère mixotrophe de la souche acétogène hydrogénotrophe, Ruminococcus hydrogenotrophicus.

Afin de déterminer si *R. hydrogenotrophicus* est capable de croître par mixotrophie (utilisation simultanée d'un substrat organique et d'un substrat inorganique), la souche est cultivée en présence de deux sources d'énergie, l'une organique, le glucose, et l'autre inorganique, H₂/CO₂. Le milieu de culture (milieu AC21 modifié comme décrit dans l'exemple 1) contient 1,4 mM de glucose et la phase gazeuse est remplacée, après ensemencement, par un mélange de H₂/CO₂ (60/40 à 156 kPa). Les cultures sont inoculées avec 0,3 ml d'une préculture de *R. hydrogenotrophicus* obtenue soit avec le glucose soit avec H₂/CO₂ comme seule source d'énergie. Au cours de l'incubation à 37°C, la consommation de gaz par la souche est suivie à l'aide de capteurs de pression fixés aux bouchons des cultures (Leclerc et al. (1997), Anaerobe 3 p307). La croissance bactérienne est estimée par mesure de la densité optique des cultures à 600 nm à l'aide d'un spectrophotomètre Spectronic 20D. Des prélèvements de surnageants de cultures sont effectués toutes les 2 heures afin d'estimer la consommation de glucose (dosage du glucose restant par méthode enzymatique Boehringer) et la production de métabolites fermentaires par dosage chromatographique (comme décrit dans l'exemple 3).

*R. hydrogenotrophicus* s'avère capable de co-utiliser les deux substrats testés. Une seule phase exponentielle de croissance de la bactérie est observée en présence des deux substrats. Lors de cette phase exponentielle, une consommation simultanée du glucose et de H₂/CO₂ est observée. Ceci témoigne du caractère mixotrophe de *R. hydrogenotrophicus* à l'égard de ces 2 substrats (Leclerc et Bernalier, soumis pour publication). En fin de phase exponentielle, le glucose est totalement consommé par la bactérie, celle-ci maintient alors son métabolisme en phase stationnaire de croissance, par l'utilisation de H₂/CO₂ restant.

La capacité de *R. hydrogenotrophicus* à co-métaboliser un édulcorant, le fructose, et H₂/CO₂, est également étudiée. La souche est cultivée sur le milieu AC21, comme décrit dans l'exemple 1, en présence de ces deux sources d'énergie. Différentes concentrations en fructose sont testées (2, 1, 0.5 et 0.25 g/l), la phase gazeuse restant composée de H₂/CO₂ (60/40, v/v, 202 kPa). Le protocole expérimental utilisé ensuite est le même que celui décrit plus haut.

*R. hydrogenotrophicus* s'avère capable de co-métaboliser le fructose et H₂/CO₂, la croissance de la bactérie étant caractérisée par une seule phase exponentielle de croissance en présence des deux substrats. Cette croissance mixotrophe est observée quelle que soit la concentration en fructose présente dans le milieu de culture et quelle que soit l'origine de l'inoculum bactérien (préculture réalisée sur fructose ou H₂/CO₂ ou fructose + H₂/CO₂). En phase stationnaire de croissance, la bactérie métabolise H₂/CO₂ uniquement, la totalité du fructose ayant été consommé.

### Exemple 7 : Effet de la lyophilisation des cultures de Ruminococcus hydrogenotrophicus sur l'expression de l'activité hydrogenotrophe

Afin de déterminer si la conservation de *R. hydrogenotrophicus* sous forme lyophilisée peut altérer sa capacité à utiliser H₂/CO₂, différentes conditions de culture, de conservations des lyophilisats, et de re-suspensions de la bactérie sont testées.

Des cultures de *R. hydrogenotrophicus* sont réalisées en fiole de 11 contenant 250 ml de milieu AC21 comme décrit dans l'exemple 1, avec du fructose (2 g/l), H₂/CO₂ (60/40, v/v, 100 kPa) ou les 2 substrats comme source d'énergie. Ces cultures sont incubées à 37°C pendant 24h, 48h ou 72h selon le(s) substrat(s) utilisé(s). Des culots bactériens sont ensuite obtenus par centrifugation (15 300 x g, 30 min, 4°C) des cultures et sont repris dans 10 ml de tampon anaérobie. Les suspensions ainsi obtenues sont aliquotées par 2 ml puis centrifugées 5 min à 14 000 x *g.* Les culots bactériens sont alors congelés à -80°C avant d'être lyophilisés pendant 1 nuit. Les lyophilisats sont conservés à 4°C sous atmosphère aérobie ou anaérobie (100% de CO₂). Après 15 à 30 jours de conservation, les lyophilisats de *R. hydrogenotrophicus* sont repris dans 5 ml de tampon de dilution anaérobie. Ces suspensions bactériennes sont alors ensemencées dans un milieu AC21 contenant H₂/CO₂ (60/40, v/v, 200 kPa) comme seule source d'énergie soit directement soit après enrichissement pendant 48h à 37°C, dans un milieu de culture complexe contenant diverses sources de carbones. Après 72h d'incubation à 37°C en présence de H₂/CO₂, la consommation de H₂ et la quantité d'acétate produite sont déterminées pour chaque culture.

La lyophilisation de *R. hydrogenotrophicus* ne semble pas affecter son potentiel hydrogénotrophe. En effet, quel que soit le substrat utilisé pour pré-cultiver la souche (fructose, H₂/CO₂ ou les 2 substrats simultanément), l'activité hydrogénotrophe de *R. hydrogenotrophicus* est restituée lors de la mise en culture des lyophilisats. De même, le mode de conservation aérobie ou anaérobie du lyophilisat influence peu l'expression du potentiel hydrogénotrophe de la bactérie. Toutefois, une activité hydrogénotrophe maximale est observée lorsque *R. hydrogenotrophicus* est pré-cultivée en présence de fructose et que le lyophilisat est conservée sous atmosphère anaérobie. De même, la culture préalable des bactéries lyophilisées sur un milieu riche en substrats organiques augmente sensiblement l'expression de leur potentiel hydrogénotrophe, quel que soit le substrat utilisé pour pré-cultiver la souche et le mode de conservation du lyophilisat. Cet effet s'explique vraisemblablement par l'augmentation de la densité bactérienne engendrée par l'enrichissement des cultures lyophilisées sur milieu complexe.

L'ensemble des résultats obtenus démontrent que la souche de *R. hydrogenotrophicus* peut être cultivée en présence de substrat organique puis conservée à 4°C sous forme lyophilisée en atmosphère aérobie ou anaérobie, sans que cela n'affecte sensiblement l'expression ultérieure de son potentiel hydrogénotrophe.

### Exemple 8 : Transfert inter-espèces de H₂, in vitro, entre bactéries fibrolytiques productrices de H₂ et R. hydrogenotrophicus.

La capacité de *R. hydrogenotrophicus* à utiliser l'H₂ produit par des espèces bactériennes fibrolytiques est étudiée *in vitro,* dans des cocultures associant la souche acétogène à une bactérie cellulolytique. Les deux espèces cellulolytiques étudiées ont été isolées au laboratoire à partir de selles humaines. Les cocultures sont réalisées sur un milieu semi-synthétique, mis au point au laboratoire, contenant une bandelette de cellulose de papier filtre Whatman n°1 comme seule source de carbone et d'énergie. Chacune des espèces cellulolytiques est ensemencée à raison de 0,5 ml d'inoculum par tube de culture. Après 48h d'incubation à 37°C, 0,5 ml d'inoculum de *R. hydrogenotrophicus* est additionné à chacune de ces cultures de bactéries cellulolytiques. Des monocultures témoins de chaque espèce cellulolytique sont effectuées parallèlement.

Une cinétique est réalisée par incubation des cultures à 37°C pendant 12 jours. Après incubation, la quantité de H₂ dans la phase gazeuse est analysée par chromatographie, la quantité de cellulose dégradée est estimée par mesure de la matière sèche restante et les produits finaux de fermentation de la cellulose sont déterminés par chromatographie en phase gazeuse et/ou par voies enzymatiques (kit Roche).

L'addition de *R. hydrogenotrophicus* à l'une ou l'autre des espèces cellulolytiques productrices de H₂ se traduit par une forte diminution de ce gaz dans les co-cultures alors qu'il s'accunnmule dans la phase gazeuse des monocultures.

*R. hydrogenotrophicus* est donc capable de ré-utiliser efficacement l'H₂ produit *in vitro* par une espèce fibrolytique lors de la fermentation de la cellulose. Ce transfert de H₂ entre *R. hydrogenotrophicus* et les espèces cellulolytiques n'entraine que peu ou pas de modification de l'activité cellulolytique des espèces fibrolytiques étudiées. En revanche, la cellulose est majoritairement fermentée en acétate dans les cocultures alors qu'elle est plutôt de type acide-mixte dans les mono-cultures (productions d'acétate, de succinate ou d'éthanol et de lactate).

### Exemple 9: Capacité de la souche acétogène hydrogénotrophe Ruminococcus hydrogenotrophicus à réduire le volume des gaz coliques in vivo, chez le rat à flore humaine.

Afin de déterminer si *R. hydrogenotrophicus* est capable de réduire le volume des gaz dans le côlon en présence d'une flore digestive complexe, *R. hydrogenotrophicus* est administré par voie orale à des rats à flore humaine et l'évolution du volume de gaz colique est suivie en mesurant l'hydrogène excrété par les voies respiratoire et rectale.

L'effet de *R. hydrogenotrophicus* est étudié en situation nutritionnelle normale et après administration d'un substrat fermentescible (lactulose) provoquant une augmentation brutale du volume de gaz dans le côlon.

Les animaux utilisés sont des rats Fischer 344 mâles, âgés de 3 mois en début d'expérience. Nés sans germe, ils sont inoculés *per os* avec 1ml d'une suspension centésimale de matières fécales humaines fraîches provenant d'un donneur adulte, non méthanogène et ayant un régime alimentaire de type occidental. L'inoculation a lieu 2 semaines avant le début de l'expérience. Les rats sont hébergés dans des isolateurs et reçoivent une alimentation semi-synthétique "de type humain" (protéines et lipides d'origines animale et végétale; glucides simples et complexes crus et cuits), stérilisée par irradiation γ à 45 kGy. Aliment et eau de boisson sont distribués *ad libitum.*

### a) Situation nutritionelle normale

L'expérience est réalisée avec 16 rats à flore humaine divisés en 2 groupes de 8, un groupe témoin et un groupe traité. Les rats du groupe traité reçoivent chaque matin pendant 28 jours, par intubation gastrique, une dose de 10⁸ à 10⁹ bactéries sous forme de 1 ml d'une culture de *R. hydrogenotrophicus* cultivée pendant 18h sur milieu AC21 (comme décrit dans l'exemple 1) glucosé (2 g/l). Le groupe témoin reçoit dans les mêmes conditions 1 ml de milieu AC21 glucosé stérile.

Après 1, 14 et 28 jours de traitement, les rats des 2 groupes sont placés pendant 12h dans des chambres respiratoires individuelles permettant la mesure de l'hydrogène excrété par voies respiratoire et rectale (voir la description des chambres respiratoires ci-après). Des échantillons d'air sont prélevés en double dans les chambres respiratoires entre 0h et 12h. La concentration en hydrogène est immédiatement déterminée par chromatographie en phase gazeuse. Les résultats des groupes témoin et traité sont comparés à l'aide d'une ANOVA pour mesures répétées.

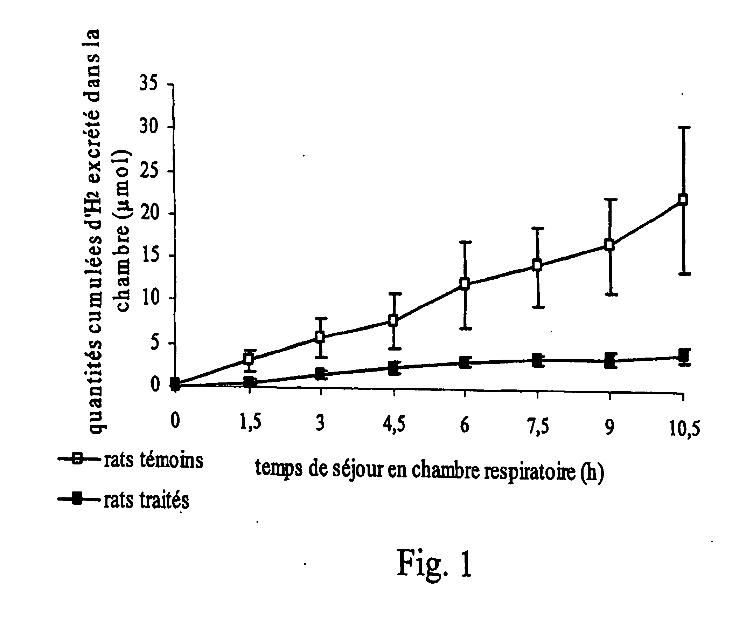

Quelle que soit sa durée du traitement, l'administration de *R. hydrogenotrophicus* diminue significativement la quantité maximale d'hydrogène excrétée (-50% à -80%) et la vitesse d'excrétion (-60% à -80%) par rapport au groupe témoin (P<0,01). Le traitement s'avère aussi efficace après 1,14 ou 28 jours (figure 1).

La flore acétogène hydrogénotrophe est dénombrée (Doré et al, 1995) dans les fèces des rats des deux groupes, à différents temps au cours de l'expérience. Dans le groupe témoin ne recevant pas *R. hydrogenotrophicus,* le niveau de la population acétogène est stable au cours du temps et comparable à celui du donneur soit environ log 6.8 acétogènes par g de fèces.

Le traitement avec *R. hydrogenotrophicus* entraîne une augmentation du niveau de la flore acétogène qui atteint alors log 7.5 /g de fèces dès 24h de traitement. Ce niveau se maintient jusqu'à la fin de l'expérience. L'augmentation de la flore acétogène chez les rats traités coïncide donc avec la diminution des quantités de H₂ excrété par ces animaux.

En fin d'expérience, les rats sont euthanasiés et autopsiés. L'administration de *R. hydrogenotrophicus* n'a pas d'effet significatif ni sur le poids corporel, ni sur le poids du foie, des reins et du caecum, ni sur le pH caecal (P>0,05). De même l'aspect macroscopique du foie et des reins des rats traités avec *R. hydrogenotrophicus* est normal et identique à celui des rats témoins.

### b) Après administration de lactulose

Cette expérience est réalisée selon le même protocole que précédemment. Le lactulose (4-O-β-D-galactopyranosyl-D-fructose), dont la fermentation dans le côlon entraine une augmentation brutale du volume de gaz, est administré par intubation gastrique, à raison de 500 mg par rat, juste avant leur passage en chambre respiratoire. L'effet de *R. hydrogenotrophicus* sur cette augmentation est examiné après 1 et 14 jours de traitement avec la souche acétogène.

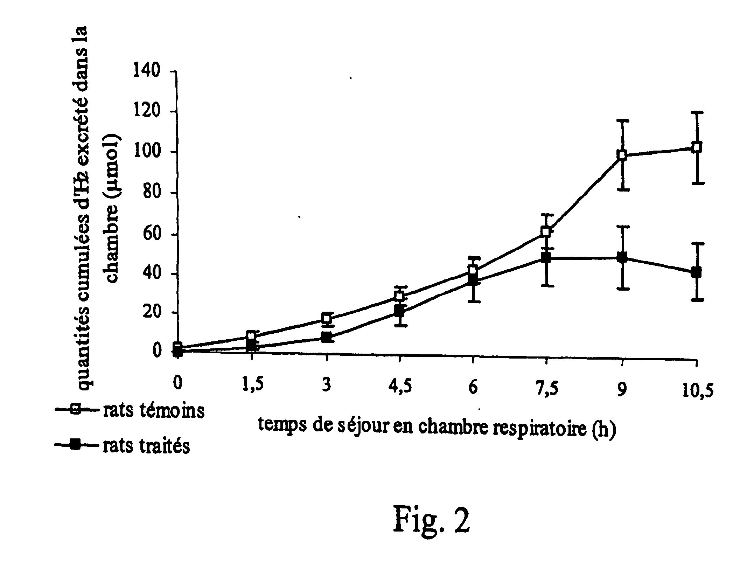

Dans ces conditions de productions excessives de gaz coliques, l'administration de *R. hydrogenotrophicus* diminue significativement la quantité maximale d'hydrogène excrétée (-40% à -50%) et la vitesse d'excrétion (-40% à -50%) par rapport au groupe témoin (P<0,05). Le traitement s'avère aussi efficace après 1 ou 14 jours (figure 2).

Dans les 2 expériences, les rats traités avec *R. hydrogenotrophicus* n'ont manifesté aucune anomalie de comportement et l'aspect et la consistance des fèces étaient identiques à ceux des rats témoins.

Les chambres respiratoires utilisées sont des enceintes autonomes étanches en polyvinyle rigide transparent, d'un volume de 30 litres, permettant d'héberger une cage contenant un animal de petite taille. Elles sont munies d'une double porte de transfert étanche (DPTE) permettant de les connecter aux isolateurs d'expérience.

Afin de préserver le statut bactérien des animaux, elles sont stérilisées avant connexion. Après transfert de l'animal de l'isolateur dans la chambre respiratoire, celle-ci est détachée de l'isolateur et connectée à un circuit fermé dans lequel l'air est poussé à l'aide d'une pompe péristaltique à travers des filtres anti-bactériens et des systèmes d'élimination du CO₂ (absorbeur contenant une solution de potasse à 40%) et de la vapeur d'eau (absorbeur contenant des cristaux de silicagel). La teneur en O₂ de l'air est mesurée par une sonde et maintenue constante à 21% à l'aide d'une électrode ampérométrique, d'un régulateur et d'une électrovanne.

Ce dispositif permet l'accumulation des gaz spécifiques des fermentations coliques (hydrogène et, le cas échéant, méthane), excrétés par voies respiratoire et rectale.

## Revendications

1. Utilisation d'au moins une souche bactérienne acétogène hydrogénotrophe non pathogène autologue pour la préparation d'une composition destinée au traitement ou à la prévention chez l'homme du syndrome de l'intestin irritable.

2. Utilisation selon la revendication 1, **caractérisée en ce que** la au moins une souche bactérienne acétogène hydrogénotrophe non pathogène est viable dans le tractus digestif.

3. Utilisation selon la revendication 1 ou 2, **caractérisée en ce que** la au moins une souche bactérienne acétogène hydrogénotrophe non pathogène appartient au genre *Ruminococcus, Clostridiun*, ou *Streptococcus*.

4. Utilisation selon la revendication 3, **caractérisée en ce que** la au moins une souche bactérienne acétogène hydrogénotrophe non pathogène appartient à l'espèce *Ruminococcus hydrogenotrophicus.*

5. Utilisation selon l'une quelconque des revendications 1 à 4, **caractérisée en ce que** la au moins une souche bactérienne acétogène hydrogénotrophe non pathogène est administrée sous une forme lui permettant d'être active dans le côlon.

6. Utilisation selon l'une quelconque des revendications 1 à 5, **caractérisée en ce** ladite composition contenant la au moins une souche bactérienne acétogène hydrogénotrophe non pathogène est destinée à être administrée par voie orale ou rectale.

7. Utilisation selon l'une quelconque des revendications 1 à 6, **caractérisée en ce que** la au moins une souche bactérienne acétogène hydrogénotrophe non pathogène est conditionnée dans un environnement anaérobie.

8. Utilisation selon l'une quelconque des revendications 1 à 7, **caractérisée en ce que** la composition comprend au moins un additif favorisant l'activité de la au moins une souche dans l'environnement digestif.

9. Utilisation selon l'une quelconque des revendications 1 à 8, **caractérisée en ce que** la composition comprend en outre au moins un autre agent actif contre au moins la pathologie visée.

10. Utilisation selon l'une quelconque des revendications 1 à 9, **caractérisée en ce que** la composition comprend en outre un support pharmaceutiquement acceptable.

## Claims

1. Use of at least one autologous nonpathogenic, hydrogenotrophic, acetogenic bacterial strain for the manufacture of a composition for treating or preventing irritable bowel syndrome in a human being.

2. Use according to Claim 1, **characterized in that** the at least one nonpathogenic, hydrogenotrophic, acetogenic bacterial strain is viable in the digestive tract.

3. Use according to Claim 1 or 2, **characterized in that** the at least one nonpathogenic, hydrogenotrophic, acetogenic bacterial strain belongs to the *Ruminococcus, Clostridium* or *Streptococcus* genus.

4. Use according to Claim 3, **characterized in that** the at least one nonpathogenic, hydrogenotrophic, acetogenic bacterial strain belongs to the species *Ruminococcus hydrogenotrophicus*.

5. Use according to any one of Claims 1 to 4, **characterized in that** the at least one nonpathogenic, hydrogenotrophic, acetogenic bacterial strain is administered in a form which allows it to be active in the colon.

6. Use according to any one of Claims 1 to 5, **characterized in that** said composition containing the at least one nonpathogenic, hydrogenotrophic, acetogenic bacterial strain is intended to be administered orally or rectally.

7. Use according to any one of Claims 1 to 6, **characterized in that** the at least one nonpathogenic, hydrogenotrophic, acetogenic bacterial strain is packaged in an anaerobic environment.

8. Use according to any one of Claims 1 to 7, **characterized in that** the composition comprises at least one additive which promotes the activity of the at least one strain in the digestive environment.

9. Use according to any one of Claims 1 to 8, **characterized in that** the composition also comprises at least one other agent active against at least the targeted pathology.

10. Use according to any one of Claims 1 to 9, **characterized in that** the composition also comprises a pharmaceutically acceptable carrier.

## Patentansprüche

1. Verwendung mindestens eines autologen acetogenen, hydrogenotrophen, nicht pathogenen Bakterienstammes für die Herstellung einer Zusammensetzung, die zur Behandlung oder Verhütung des Reizkolonsyndroms beim Menschen bestimmt ist.

2. Verwendung nach Anspruch 1, **dadurch gekennzeichnet, dass** der mindestens eine acetogene, hydrogenotrophe, nicht pathogene Bakterienstamm im Verdauungstrakt lebensfähig ist.

3. Verwendung nach Anspruch 1 oder 2, **dadurch gekennzeichnet, dass** der mindestens eine acetogene, hydrogenotrophe, nicht pathogene Bakterienstamm der Gattung *Ruminococcus, Clostridium* oder *Streptococcus* angehört.

4. Verwendung nach Anspruch 3, **dadurch gekennzeichnet, dass** der mindestens eine acetogene, hydrogenotrophe, nicht pathogene Bakterienstamm der Art *Ruminococcus hydrogenotrophicus* angehört.

5. Verwendung nach irgendeinem der Ansprüche 1 bis 4, **dadurch gekennzeichnet, dass** der mindestens eine acetogene, hydrogenotrophe, nicht pathogene Bakterienstamm in einer Form verabreicht wird, die ihm ermöglicht, im Kolon aktiv zu sein.

6. Verwendung nach irgendeinem der Ansprüche 1 bis 5, **dadurch gekennzeichnet, dass** die Zusammensetzung, welche den mindestens einen acetogenen, hydrogenotrophen, nicht pathogenen Bakterienstamm enthält, dazu bestimmt ist, auf oralem oder rektalem Weg verabreicht zu werden.

7. Verwendung nach irgendeinem der Ansprüche 1 bis 6, **dadurch gekennzeichnet, dass** der mindestens eine acetogene, hydrogenotrophe, nicht pathogene Bakterienstamm in einer anaeroben Umgebung abgepackt wird.

8. Verwendung nach irgendeinem der Ansprüche 1 bis 7, **dadurch gekennzeichnet, dass** die Zusammensetzung mindestens ein Additiv umfasst, welches die Aktivität des mindestens einen Stammes in der Verdauungsumgebung begünstigt.

9. Verwendung nach irgendeinem der Ansprüche 1 bis 8, **dadurch gekennzeichnet, dass** die Zusammensetzung darüber hinaus mindestens einen anderen Wirkstoff gegen mindestens die betreffende Krankheit umfasst.

10. Verwendung nach irgendeinem der Ansprüche 1 bis 9, **dadurch gekennzeichnet, dass** die Zusammensetzung darüber hinaus einen pharmazeutisch annehmbaren Träger umfasst.
